Europäisches Patentamt

European Patent Office

Office européen des brevets

(11) Publication number: **0 269 304**
**A1**

# (12) EUROPEAN PATENT APPLICATION

(21) Application number: 87309907.1

(51) Int. Cl.⁴: **G01N 33/52**

(22) Date of filing: 10.11.87

(30) Priority: 10.11.86 US 929233

(43) Date of publication of application:
01.06.88 Bulletin 88/22

(84) Designated Contracting States:
CH DE FR GB IT LI SE

(71) Applicant: EASTMAN KODAK COMPANY
Patent Department 343 State Street
Rochester New York 14650(US)

(72) Inventor: Helfer, Jeffrey Lawrence EASTMAN
KODAK COMPANY
Patent Department 343 State Street
Rochester New York 14650(US)

(74) Representative: Davis, Ian Ellison et al
Kodak Limited Patent Department Headstone
Drive
Harrow Middlesex HA1 4TY(GB)

(54) Disposable test device.

(57) There is disclosed a test device for storing and dispensing a liquid. The device comprises

means (12, 12a, 12b) for supporting a test element within said device;

means (30, 30a, 30b) for storing such liquid within said device for an extended period of time,

primary dispensing means, including a wick (36, 36'; 36a, 36'a; 36b, 36'b), for dispensing the liquid onto an absorbing surface of such test element;

hinge means (40, 40a, 40b) for permitting the supporting means and the storing means to move relative to each other, between a first spaced apart position, and a second position wherein the dispensing means contacts such absorbing surface of such test elements;

and removable cover means (29, 29a, 29b) for inhibiting evaporation of such liquid during storage of the device, and/or patient sample added to the test element.

FIG. I

## DISPOSABLE TEST DEVICE

This invention concerns disposable test devices for storing and dispensing liquids onto one or more test elements.

In the field of clinical assays, the trend has been to eliminate the use of liquids such as reagents as much as possible. With such assays, all that is needed is an aliquot of a patient sample, which is added to a test element bearing the necessary reagents in dried form. Examples of useful test elements that permit such a technique are included in US-A-3992158.

However, in some assays, notably immunoassays, it has not been possible to totally eliminate the use of liquids. In these assays, part of the reagents are stored dry, but a liquid wash step is used, and additional reagents may be added in liquid form to complete the development of a detectable change that is detected visually or in an analyzer. A problem in such cases has been to develop a disposable device that provides for the storage and use of such liquids, in a manner that minimizes the action to be taken by the operator. That is, if untrained personnel, such as a patient, are to operate such devices, any liquid reagent should be provided in such a way that there is no need for the operator to carefully measure, and then apply, the liquid reagent to an appropriate test element. For example, test kits that require a patient to measure out a teaspoon of reagent, and then carefully pour it onto a test element are subject to substantial error, both in the measurement and in the application of the reagent. Successful solutions that avoid this problem of careful measurement and application will thus make available test kits that are more useful by patients as home test kits.

Such operational difficulties are even more pronounced in an immunoassay using a radial wash step, since the rate of transfer of wash liquid to the element is critical. If the proper rate is not used, the test becomes inaccurate.

Because such test kits are subject to extended periods of storage, additional problems have to be dealt with. That is, the liquids have to be stored in a way that preserves them against loss by evaporation. Otherwise, the liquid needed for a wash step may be totally missing. Furthermore, since aging will likely affect the ability of the reagents to react, a correcting factor has to be added that will automatically compensate for such aging.

## SUMMARY OF THE INVENTION

In accord with the invention, the above-noted problems have been solved by a disposable test device for delivering stored liquid to a contained test element having an absorbing surface. The device comprises means for supporting the test element within the device,

means, including a storage chamber, for storing such liquid within the device,

primary dispensing means, including a wick, for dispensing the liquid out of the storing means upon contact with the absorbing surface of such test element;

hinge means for permitting the supporting means and the storing means to move relative to each other, between a first spaced-apart position, and a second position wherein the dispensing means contacts such absorbing surface of such test elements,

and removable cover means for inhibiting evaporation of a) such liquid during storage of the device, and/or b) patient sample added to a contained test element.

Thus, it is an advantageous feature of the invention that a disposable test device is provided that will allow the use of liquids without requiring care in the measurement or application of such liquids.

It is another advantageous feature of the invention that such a disposable test device permits storage of the liquid over an extended period of time, with no adverse results.

Yet another advantageous feature of the invention is that such a disposable device can be constructed to prevent unwanted evaporation of patient sample from the test element, by the same means as prevents evaporation of the liquid.

It is a related additional advantageous feature of the invention that such a disposable device compensates automatically for any degradation in detectable signal caused by aging of the device.

The present invention will now be described by way of example with reference to the accompanying drawings in which:

Fig. 1 is an isometric view of a test device constructed in accord with the invention, the cover having been hinged out of the way prior to dispensing the stored liquid reagents;

Figs. 2 and 3 are fragmentary sectional views taken generally perpendicularly through the plane of the reagent storage compartment along the lines II-II and III-III of Fig. 1, respectively;

Fig. 4 is a sectional view taken through the middle of yet another embodiment of the invention, shown in its storage mode;

Fig. 5 is a sectional view similar to that of Fig. 4, but showing the device in its liquid reagent dispensing mode;

Fig. 6 is a sectional view taken generally along the line VI-VI of Fig. 4;

Fig. 7 is a sectional view taken generally along the line VII-VII of Fig. 5; and

Fig. 8 is a sectional view similar to that of Fig. 4, but of still another embodiment of the invention.

This invention is described hereinafter primarily in connection with a preferred embodiment of a test device for detecting infectious diseases. In addition, the invention is useful in a device that must store and apply a liquid to a test element for any purpose, regardless of what is being detected or assayed.

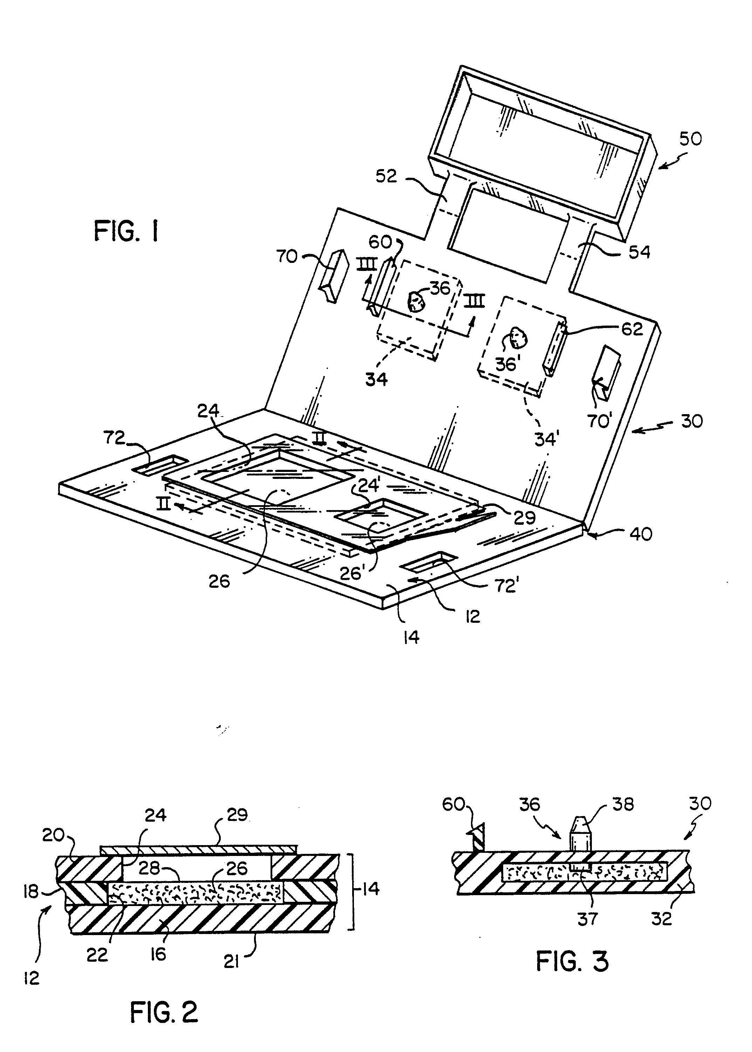

In the embodiment of Fig. 1, a device constructed in accord with the invention comprises a portion 12 that supports a test element, a portion 30 that mounts and stores a confined liquid, and hinge means 40 that permits portions 12 and 30 to be moved relative to each other from a spaced apart first position shown in Fig. 1, to a second position in which the dispensing wicks hereinafter described touch their respective test elements. Portion 12 comprises a generally planar frame 14, Fig. 2, which in turn is preferably a laminate of layers 16, 18 and 20. Most preferably, surface 21 of layer 16 is a polished surface to allow ready viewing of the test element underneath it, from a position exterior to surface 21. Layers 18 and 20 are formed with openings 22 and 24, sized to allow a primary test element 26 having an exterior surface 28, to be supported within opening 22. Most preferably, such storage of the test element is permanent, that is, it does not require the user to put the test element in or on frame 14. Opening 24 is sized to allow the patient sample as well as stored liquid to be applied to surface 28 of element 26. Any test element 26 can be mounted within opening 22, containing one or more useful dried reagents in one or more layers. Surface 28 is preferably that of a spreading layer, such as that described in the aforesaid US-A-3992158.

To protect element 26 from contamination and/or from excessive moisture or excessive drying of the patient sample, a resealable, strippable protective layer 29, preferably of a metallic foil, is disposed over opening 24 and held in place by a suitable adhesive, not shown. For ease of illustration, layer 29 is shown as being transparent. It is stripped back by the user at the time of use, and resealed after the sample is placed on the element. Frame 12 also includes an opening 24' spaced

laterally away from opening 24, Fig. 1, under which is disposed a reference element 26' held in an opening corresponding to opening 22 for the test element 26. Preferably, opening 24' is smaller than 24 to allow the user to recognize it as the reference element and thus not the test element. Reference element 26' has the same chemistry as test element 26, and can be simply an extension thereof as is readily accomplished by extending opening 22 to underlie both opening 24 and opening 24'.

Turning now to portion 30, Figs. 1 and 3, this comprises a generally planar frame 32 within which cavities 34 and 34' are formed to hold the liquid, Figs. 1 and 3. Wicks 36 and 36' are mounted in the frame, one end 37 of which projects into the liquid. The opposite end 38 is configured, such as into a point, to allow ready wicking of the liquid reagent out of the wick when it contacts a surface.

The liquids within cavities 34 and 34' are preferably identical, except that cavity 34' also may include a predetermined concentration of the analyte under test. This concentration is selected to be about mid-way between the concentration that is normally detected as a positive value, and that which is considered to be a negative value. In some cases, no analyte need be present in the liquid for the reference element.

Alternatively, elements 26 and 26' can be two separate elements, not shown, with element 26' containing such predetermined concentration of analyte. In such a case, the two liquids in cavities 34 and 34' are preferably identical in composition, that is, they are simply water for a radial wash step.

To protect wicks 36 and 36' from contamination and large-scale evaporation of the liquid, a removable cover 50, shown in Fig. 1 with a 5-sided box shape, is attached to portion 30 through two hinge arms 52 and 54. These arms preferably are living hinges, which allows cover 50 to close over wicks 36 and 36' in contact with frame 32, not shown, or to be removed to the position shown in Fig. 1. To removably hold cover 50 in such covering configuration and to assure a gas tight seal, ridges 60 and 62 protrude from frame 32, as shown in greater detail in Fig. 3, to frictionally engage the inside of cover 50.

To allow portions 12 and 30 to be held together in their second position wherein wicks 36 and 36' contact elements 26 and 26' respectively, and to meter the liquid onto those elements, locking means are provided, Fig. 1. These comprise two latch posts 70 and 70', in one of the portions 12 and 30, and corresponding latch openings 72 and 72' in the other portion.

To use device 10, the user simply peels back layer 29. An aliquot of the patient's liquid, e.g., a drop of whole blood, urine or the like, is placed onto test element 26 only. Layer 29 is resealed for about three to seven minutes to allow interaction of the patient sample with the test element. Layer 29 is then removed entirely, and cover 50 is hinged away from wicks 36 and 36'. Portions 12 and 30 are hinged together to bring wicks 36 and 36' into contact with their respective elements, and latch posts 70 and 70' latch into openings 72 and 72'. After a second length of time, element 26 is then read, either by visual comparison against a standard, or in an analyzer, through surface 21 to detect the concentration of analyte present. If the concentration, as evidenced by a density build-up, for example, is greater than the reference density produced in reference element 26', then the test is positive. After element 26 is read, the device is disposed of.

Useful tests for the device of this invention include any immunoassay that requires delivery of a liquid, such as water, during a radial wash step that separates free labeled antigen from bound labeled antigen. In addition, the liquid and thus the radial wash may include a reagent, such as a substrate for an enzyme label kept in the dried element. As used herein, "reagent" is any compound or solute that is required in the test element in order to produce a detectable change that is proportional to the amount of analyte being detected. In the aforementioned immunoassay reactions, the reagent may be added no sooner than during radial wash to insure it does not interfere with the complexing of the labeled antigen with the immobilized antibody.

Alternatively, the liquid in the cavities may be only the solvent, e.g., water. Assays that are useful in such an arrangement are those which use, for example, fluorescent labels or other labels that do not require a rate of consumption determination. Examples of such labels are well-known in the art.

The following is a specific example of a useful immunoassay that one skilled in such art will recognize can be conducted with the device of the invention.

EXAMPLE DIGOXIN TEST ELEMENT

In this example, the liquid stored in the cavity includes as a reagent, the substrate for the enzyme label that is in the test element.

The test element is coated to have the following layers:

| | | | |
|---|---|---|---|
| Spreading Layer | Poly(vinyltoluene-co-p-t-butylstyrene-co-methacrylic acid) beads | 50–300 | $g/m^2$ |
| | Poly(methylacrylate-co-2-acrylamido-2-methylpropane sulfonic acid-co-2-acetoacetoxyethyl methacrylate) adhesive | 1–10 | $g/m^2$ |
| | 2-(3,5-Dimethoxy-4-hydroxyphenyl)-4,5-bis(4-dimethylaminophenyl)imidazole leuco dye | 0.01–1 | $g/m^2$ |
| | Staphylococcus aureus coated with digoxin antibodies | 0.2–5 | $g/m^2$ |
| | SURFACTANT 10G surfactant | 0.1–10 | $g/m^2$ |
| | Dimedone antioxidant | 0.005–0.5 | $g/m^2$ |
| Water-Soluble Layer | Poly(vinyl alcohol) | 0.1–10 | $g/m^2$ |
| | ZONYL FSN surfactant | 0.01–1 | $g/m^2$ |
| | Potassium phosphate buffer (pH 7) | 0.01–1 | $g/m^2$ |
| | Digoxin-peroxidase conjugate | $10^{-6}$–$10^{-4}$ | $g/m^2$ |
| Reagent Layer | Gelatin (hardened) | 1–100 | $g/m^2$ |
| | SURFACTANT 10G surfactant | 0.02–2 | $g/m^2$ |
| | Potassium phosphate buffer (pH 7) | 0.05–5 | $g/m^2$ |
| | α-Glycerol phosphate oxidase | 200–20,000 | $I.U./m^2$ |
| | 4-Hydroxyacetanilide | 0.01–1 | $g/m^2$ |

Poly(ethylene terephthalate) Support

The patient pricks his finger and spots the drop of blood onto the exposed surface of the spreading layer, and reseals the foil layer over the test element to prevent evaporation off the sample. After five minutes, the foil cover layer is removed entirely, and the device closed and latched with the wicks in contact with elements 26 and 26', Fig. 1. During those five minutes, the liquid drop of blood does two things - it causes the digoxin labeled with peroxidase, as well as the α-glycerol phosphate oxidase to migrate from the second and lower layers, respectively, to the upper layer, and provides unlabeled digoxin from the patient that competes with the labeled digoxin for the digoxin antibodies in the upper layer.

The liquid of the cavities is, in such a case, 10 μl of water that contains as the substrate for the glycerol phosphate oxidase, 100 mmole of α-glycerol phosphate.

Because of the cover and construction of wicks 36 and 36', and their cavities 34 and 34', device 10 provides effective protection to the liquid reagents for extended storage periods. In addition, application of the reagents to the test and reference elements automatically follows hinging of the two portions 12 and 30 together, so as to minimize possible error on the part of the user.

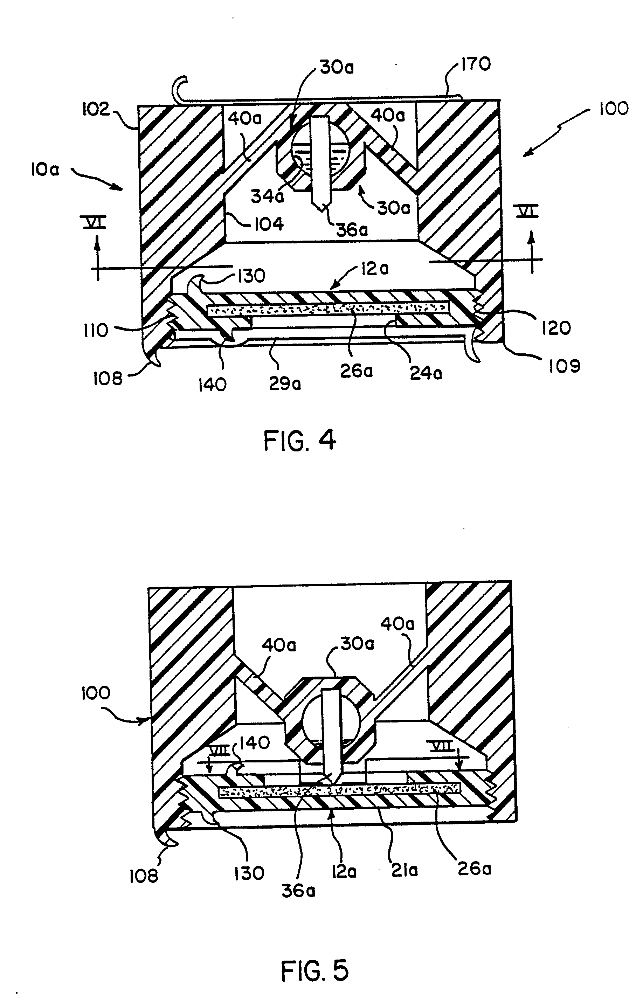

It is not necessary that cover 50 be a part separate from, e.g., portion 12. Instead, the two can be one piece, as shown in the embodiment of Figs. 4-7. Parts similar to those previously described bear the same reference numeral to which the distinguishing suffix "a" has been added.

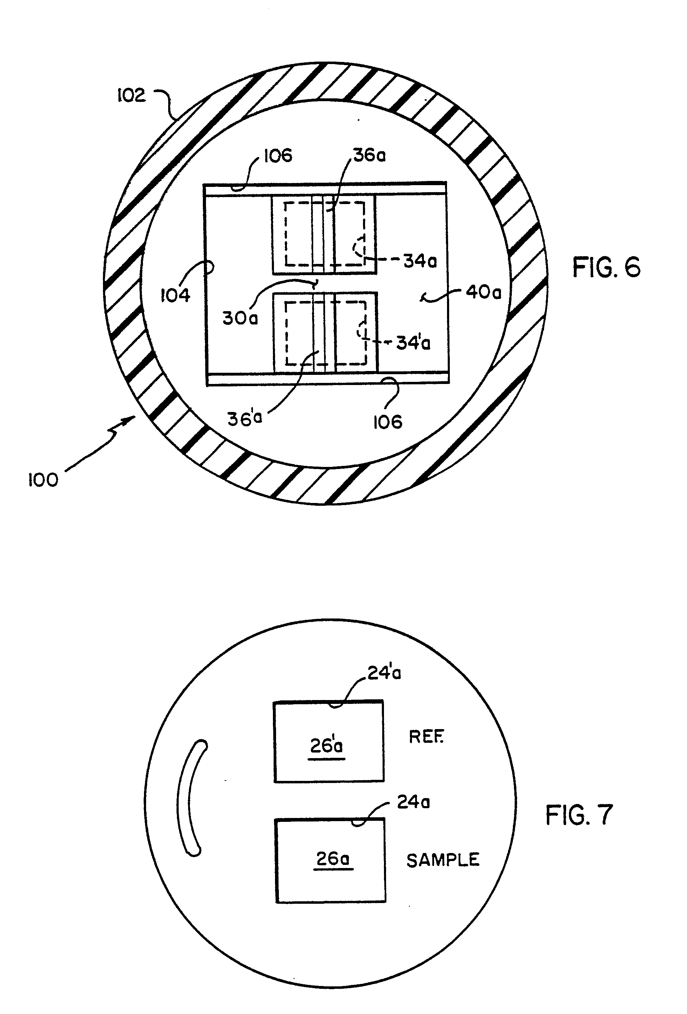

Thus, device 10a comprises a portion 30a within which is provided two cavities 34a and 34'a, Fig. 6, out of which project respective wicks 36a and 36'a, as in the previous embodiment. Portion 30a is in turn connected, via hinges 40a, Fig. 4, to a member 100 within which is removably located the test elements and reference element. Hinges 40a differ from hinges 40 of the previous embodiment in that hinges 40a are bistable. Member 100 is cylindrical on the outside surface 102, but rectangular on its inside surface 104, Fig. 6, facing portion 30a. Clearance is preferably provided between portion 30a and end walls 106 of inside surface 104, for hinging movement of portion 30a. An identifying ridge 108 is provided at one end 109 of member 100, to allow the user to properly orient portion 12a as is hereinafter described.

Unlike the previous embodiment, the mounting portion 12a for the test and reference elements also comprises the protective cover for wicks 36a and 36'a. Thus, mounting portion 12a comprises a circular laminate, Figs. 4 and 7, within which are held test element 26a and reference element 26'a, in the manner described in the previous embodiment. portion 12a is provided with a protective layer 29a, Fig. 4, as before, but differs from the previous embodiment in that it has male threads 110 around its circumference. The interior of frame 100 is female threaded at 120 to mate with threads 110. Grasping lips 130 and 140 are also provided to allow portion 12a to be grasped and rotated. Openings 24a and 24'a can be labeled as "SAMPLE" and "REF.", respectively, Fig. 7, if they are equal in size, or they can be unequal in size as in the previous embodiment, with or without labels.

Optionally, a layer 170 of protective foil is adhered on member 100 outside of portion 30a, to further inhibit evaporation of liquid reagents out of wicks 36a and 36'a.

Operation of this embodiment will be readily apparent from the preceding description. As shown in Figs. 4 and 5, layer 29a is peeled off, as is layer 170, if used. Until this point, the plastic-to-plastic seal provided by threads 110 and 120 provides excellent protection to wicks 36a and 36'a, and inhibition of evaporation, allowing an extended period of storage. Upon exposure of test element 26a, the patient sample is applied to the test element only.

Portion 12a is then grasped, using lip 140, and unscrewed. It is then flipped over, and rescrewed into member 100, Fig. 5, using lip 130. The alignment of lip 130 with ridge 108 is an indication to the user that openings 24a and 24'a are properly aligned with their wicks. Or alternatively, a rotation limiter such as a quarter-turn luer fitting, not shown, can be used. In either case, the patient sample incubates in layer 26a, Fig. 5, a desired length of time. Next, portion 30a is pushed downward. Because hinges 40a are bistable hinges, they will flip into the only other stable state, which is with wicks 36a and 36'a in contact with their respective elements 26a and 26'a. The results of the test are read through polished surface 21a, Fig. 5, as in the previous embodiment.

It will be readily appreciated that the device of this embodiment has the advantage that the same protective cover 12a serves to protect from evaporation not only the reagents in cavity 34a, but also the sample from element 26a.

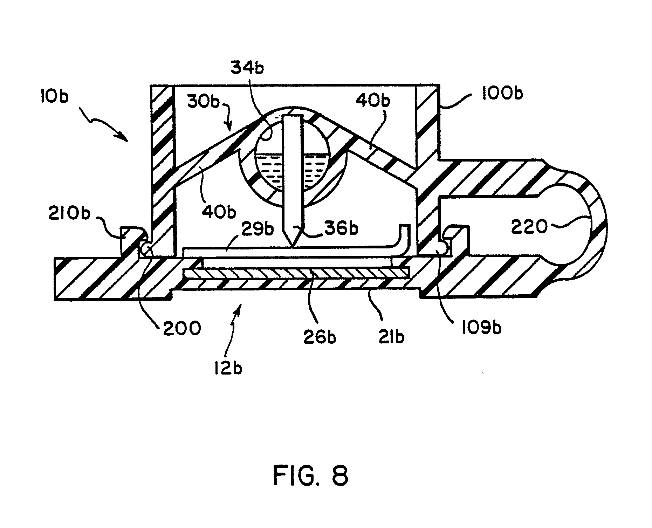

Alternatively, Fig. 8, the means for mounting the test elements can be a cap that snaps, rather than screws, into place as the cover that inhibits evaporation of patient sample or the liquid from the cavities. Parts similar to those previously described bear the same reference numerals, to which the distinguishing suffix "b" has been appended. Thus, cylindrical member 100b has a portion 30b that is hinged at 40b to the member, a wick 36b in portion 30b being useful to dispense each liquid out of its respective cavity 34b as described in the previous embodiment. However, portion 12b, though acting as a cover as before, during storage of device 10b, snaps into place. Foil 29b is placed over element 26b as before, but in this embodiment, it is to prevent moisture from the wicks affecting the element. The structure which permits snap-locking is a lip 200 extending outwardly around the circumference at end 109b, and a corresponding snap ridge 210b on portion 12b that mates with lip 200. Most preferably, portion 12b is integrally connected to member 100b by a living hinge 220.

To use this embodiment, portion 12b is unsnapped and the patient sample is spotted onto the primary test element 26b. Thereafter, portion 12b is snapped back into place on lip 200. After a suitable incubation period, portion 30b is pushed downwardly to its other bistable position, so that the pair of wicks dispense the liquid from the two cavities as described above, onto test element 26b and the reference element, not shown. Reading is accomplished through the exterior surface 21b as in the previous embodiment.

'SURFACTANT 10G' and 'ZONYL FSN', referred to above, are trade marks.

## Claims

1. A disposable test device for delivering stored liquid to a contained test element having an absorbing surface constructed to receive a patient sample, comprising

　　means for supporting the test element within said device,

　　　means, including a storage chamber, for storing such liquid within said device,

　　　primary dispensing means, including a wick, for dispensing said liquid out of said storing means upon contact with the absorbing surface of such test element;

　　　hinge means for permitting said supporting means and said storing means to move relative to each other, between a first spaced-apart position, and a second position wherein said dispensing means contacts such absorbing surface of such test elements,

　　　and removable cover means for inhibiting evaporation of a) such liquid during storage of said device, and/or b) patient sample added to a contained test element.

2. A device as defined in claim 1, wherein said hinge means is a flexible hinge integrally formed with both said storing means and said supporting means.

3. A device as defined in claim 1, wherein said supporting means is part of said removable cover means.

4. A device as defined in claim 1 or 3, and further including a cylindrical frame, said storing means being integrally connected to said frame through said hinge means,

　　　and wherein said supporting means comprises a cap in which such test element is mounted, said cap and said frame being provided with means for engaging each other.

5. A device as defined in claim 4, wherein said cap is integrally connected to said frame through a hinge.

6. A device as defined in any one of the preceding claims, and further including means for holding said supporting means and said storing means in said second position.

7. A device as defined in claim 1, 3, 4, 5 or 6 wherein said hinge means comprises a bistable hinge integrally formed with said storing means.

8. A device as defined in claim 1, and further including a reference element mounted within the device, and auxiliary means for dispensing said liquid onto said reference element.

9. A device as defined in claim 8, wherein said auxiliary dispensing means is separate from and mounted adjacent to said primary dispensing means, and wherein said hinge means permits said primary and said auxiliary dispensing means to dispense said liquid simultaneously onto their respective elements.

FIG. I

FIG. 2

FIG. 3

FIG. 4

FIG. 5

FIG. 6

FIG. 7

FIG. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 182 373 (BOEHRINGER MANNHEIM GmbH) <br> * Page 2, lines 1-16; page 8, lines 9-19; page 13, lines 1-14 * <br> --- | 1-3,5,7 ,8 | G 01 N 33/52 |
| X | DE-A-3 130 749 (BOEHRINGER MANNHEIM GmbH) <br> * Page 1, lines 1-25; page 5, line 29 - page 10, line 33; page 15, lines 1-7; figures 1-5 * <br> --- | 1-3,5,7 ,8 | |
| X | US-A-3 933 594 (T.W. MILLIGAN et al.) <br> * Column 1, lines 28-47; column 3, lines 26-35; column 3, lines 56-63; column 5, line 57 - column 6, line 57 * <br> --- | 1-3,5,7 ,8 | |
| Y | US-A-3 996 006 (J.E. PAGANO) <br> * Column 1, lines 26-39,42-52,55-63; column 2, line 66 - column 4, line 17 * <br> --- | 1-3,5,7 -9 | |
| Y | GB-A-1 277 193 (WESTINGHOUSE ELECTRIC CORP.) <br> * Page 2, lines 62-82; page 5, line 73 - page 6, line 29 * <br> ----- | 1-3,5,7 -9 | TECHNICAL FIELDS SEARCHED (Int. Cl.4) <br><br> G 01 N |

The present search report has been drawn up for all claims

| Place of search | Date of completion of the search | Examiner |
|---|---|---|
| THE HAGUE | 24-03-1988 | VAN BOHEMEN C.G. |

EPO FORM 1503 03.82 (P0401)